(19) Europäisches Patentamt
European Patent Office
Office européen des brevets

(11) Veröffentlichungsnummer: **0 015 231**
**B1**

(12) # EUROPÄISCHE PATENTSCHRIFT

(45) Veröffentlichungstag der Patentschrift: **01.09.82**

(21) Anmeldenummer: **80810018.4**

(22) Anmeldetag: **24.01.80**

(51) Int. Cl.³: **C 07 C 62/38**, C 07 C 51/347, C 07 C 51/353, C 07 C 51/083, C 07 C 51/087, C 07 C 69/757, C 07 C 67/343, C 07 C 67/08, C 07 C 67/29, C 07 D303/16, C 08 F 8/14

(54) **Indenoncarbonsäuren und Ester derselben sowie Verfahren zu ihrer Herstellung.**

(30) Priorität: **30.01.79 CH 885/79**

(43) Veröffentlichungstag der Anmeldung: **03.09.80 (Patentblatt 80/18)**

(45) Bekanntmachung des Hinweises auf die Patenterteilung: **01.09.82 Patentblatt 82/35**

(84) Benannte Vertragsstaaten: **CH DE FR GB**

(56) Entgegenhaltungen: **FR M 3 340**

(73) Patentinhaber: **CIBA-GEIGY AG**
***Patentabteilung Postfach***
**CH-4002 Basel (CH)**

(72) Erfinder: **Bosshard, Hans**
**Hohe Winde-Strasse 23**
**CH-4059 Basel (CH)**
Erfinder: **Rempfler, Hermann, Dr.**
**Brückilsmattstrasse 16**
**CH-4107 Ettingen (CH)**
Erfinder: **Zweifel, Hans, Dr.**
**Lothringerstrasse 93**
**CH-4056 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 015 231 B1

## Indenoncarbonsäuren und Ester derselben sowie Verfahren zu ihrer Herstellung

Die vorliegende Erfindung betrifft Halogenindenoncarbonsäuren, Verfahren zu deren Herstellung sowie Ester dieser Halogenindenoncarbonsäuren. Die Halogenindenoncarbonsäuren gemäss dieser Erfindung eignen sich für die Herstellung von unter dem Einfluss von Licht vernetzbaren Polymeren. Lichtempfindliche Polymere auf der Basis von Halogenindenoncarbonsäuren waren bisher nicht bekannt und auch eine entsprechende Lichtempfindlichkeit dieser Säure selbst ist nicht beschrieben.

Es ist jedoch bereits eine ganze Reihe von andersartigen durch Lichteinwirkung vernetzbaren Polymeren bekannt, bei denen die Vernetzung durch eine Photocyclodimerisierung von spezifischen C = C-Doppelbindungen bewirkt wird. Als Stand der Technik, welcher sowohl derartige lichtempfindliche Polymere als auch entsprechende lichtempfindliche Monomere, welche für die Herstellung von solchen Polymeren geeignet sind, umfasst, sind die folgenden Patentanmeldungen bzw. Patente anzuführen :

Die japanischen Offenlegungsschriften :

JA 49-128991, JA 49-128992, JA 49-128993, JA 50-5376, JA 50-5377, JA 50-5378, JA 50-5379, JA 50-5380, JA 50-9682, JA 50-10884, JA 50-77363.

Die Deutschen Offenlegungsschriften :

DE 2 031 573, DE 2 032 037, DE 2 626 795, DE 2 407 033.

Das US-Patent 3 079 041.

Bei diesen bekannten lichtempfindlichen Polymeren handelt es sich mit Ausnahme von denen gemäss DE 2 407 033 um Substanzen, bei denen die Lichtempfindlichkeit auf Maleinimidgruppen oder Abkömmlingen davon beruht. Dagegen beruht die Lichtempfindlichkeit der Polymeren nach DE 2 407 033 auf substituierten 1-Carbonyloxy-1H-naphthalin-2-on-Gruppen.

Die vorbekannten vernetzbaren Polymeren haben den Nachteil einer relativ geringen photochemischen Empfindlichkeit, weshalb sie sich nicht oder nur schlecht für zahlreiche Anwendungen eignen, für die hochlichtempfindliche Substanzen benötigt werden, oder aber sie erfordern die Mitverwendung von bekannten Photosensibilisatoren, wie Benzophenon, Thioxanthon und dergleichen. Im übrigen sind dieselben farblos. Häufig ist es aber aus technischen Gründen erwünscht, dass das lichtempfindliche Material eine spezifische Farbe aufweist. Bei den bekannten Polymeren wird dann eine aufwendige Einfärbung erforderlich, diemanchmal auch noch zu Störungen führt.

In einer Publikation von Th. Zincke und M. Engelhardt, Annalen d.Ch. *283* (1894) 341-360, ist bereits die Chlorindenoncarbonsäure der Formel

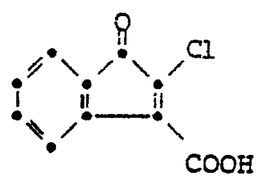

beschrieben worden.

Die Herstellung dieser Verbindung erfolgt nach einem sehr umständlichen, mehrstufigen Verfahren, welches das Produkt offensichtlich in schlechter Ausbeute liefert. Die Herstellung irgendwelcher anderer Chlor-indenoncarbonsäuren, beispielsweise solcher, bei denen H-Atome des Sechsrings obiger Formel substituiert sind, gelingt, vermutlich aufgrund von Nebenreaktionen wie Oxydation, nicht.

R.E. Lutz hat im J. Am. Chem. Soc. *52* (1930) 3404-3422, u.a. über die Friedel-Crafts-Reaktion von Dibrommaleinsäureanhydrid mit Benzol in Gegenwart von $AlCl_3$ berichtet. Er erhielt vorwiegend harzige hochmolekulare Produkte und in geringer Ausbeute auch die Brom-carbonsäure der Formel

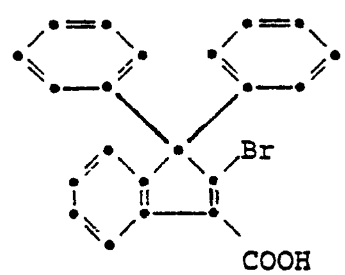

In einer weiteren Publikation « A convenient synthesis of some highly functionalized chromones and indones », Synthetic Communications *9* (2), 129-139 (1979), haben G. Roberge und P. Brassard über Indenoncarbonsäuren berichtet, welche am Sechsring in 7-Stellung eine OH-Gruppe enthalten. Diese Verbindungen sind jedoch gänzlich ungeeignet für die Herstellung von unter dem Einfluss von Licht vernetzbaren Polymeren.

Die Aufgabe der Erfindung besteht darin, ein Verfahren zur Herstellung der Halogen-indenoncarbonsäure der oben angegebenen Formel zu finden, welches unkompliziert ist und sich technisch in möglichst einfacher und wirtschaftlicher Weise realisieren lässt, welches zu hoher Ausbeute und von vornherein zu einem möglichst reinen Produkt führt und welches es ausserdem möglich macht, bei Wechsel der Ausgangsprodukte auch andere, d.h. substituierte Halogenindenoncarbonsäuren herzustellen. Somit ist ein Teil der Aufgabe der Erfindung auch die Bereitstellung von solchen neuen substituierten Halogen-indenoncarbonsäuren, welche wie die unsubstituierten Säuren u.a. für die Herstellung von unter dem Einfluss von Licht vernetzbaren Polymeren geeignet sind.

Die Aufgabe der Erfindung umfasst aber auch die Bereitstellung von Estern dieser Halogenindenoncarbonsäuren, welche endständig jeweils eine reaktive Gruppe enthalten, die polymerisierbar und/oder für die Reaktion mit seitenständigen reaktiven Gruppen von Polymeren geeignet ist; welche letztlich vorteilhaft als Ausgangsprodukte für die Herstellung von unter Lichteinfluss vernetzbaren Polymeren einsetzbar sind.

Die Halogen-indenoncarbonsäuren sowie die entsprechenden Ester sollen gemäss der Aufgabe der Erfindung Ausgangsprodukte für solche unter dem Einfluss von Licht vernetzbare Polymere sein, welche in ihren Eigenschaften den lichtempfindlichen Polymeren des Standes der Technik überlegen sind. Diese Polymeren sollen nämlich eine ganz spezifische Eigenfärbung aufweisen, welche bei den Polymeren des Standes der Technik nicht auftritt.

Gleichzeitig sollen sie aufgrund hoher Eigenabsorption im langwelligen UV ohne Einsatz von Sensibilisatoren leicht vernetzen.

Gegenstand der Erfindung ist ein Verfahren zur Herstellung von Indenoncarbonsäuren der Formel I

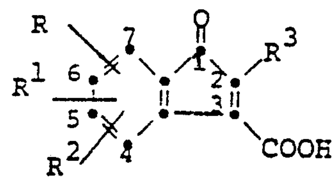

(I)

worin

$R^3$ für Cl oder Br, vorzugsweise für Cl, steht und

R, $R^1$ und $R_2$ gleich oder verschieden sind,

R einen n-Alkylrest mit 1 bis 4 C-Atomen, vorzugsweise $CH_3$, oder aber H, Cl, Br oder F,

$R^1$ einen n-Alkylrest mist 1 bis 4 C-Atomen, vorzugsweise $CH_3$, oder aber H,

$R^2$ H oder $CH_3$ oder aber

$R^1$ und $R^2$ zusammen die Gruppe $—CH_2CH_2CH_2—$ bedeuten, wobei im letzteren Fall die Bindung an den Sechsring über die C-Atome in 5- und 6-Position des Kerns erfolgt, welches dadurch gekennzeichnet ist, dass man eine aromatische Verbindung der Formel II

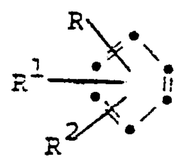

(II)

in der an mindestens 2 benachbarten C-Atomen des Kerns die H-Atome nicht substituiert sind und in der R, $R^1$ und $R^2$ dieselbe Bedeutung wie in Formel I haben, entweder zusammen mit einem Dihalogenmaleinsäureanhydrid der Formel III

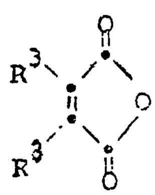

(III)

oder zusammen mit einem Ester der Formel IV

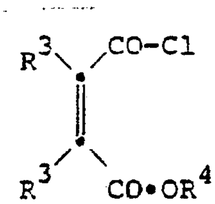

(IV)

wobei $R^3$ dieselbe Bedeutung wie in Formel I hat und $R^4$ einen niedermolekularen organischen Rest, insbesondere einen aliphatischen, einen cycloaliphatischen, einen aliphatischaromatischen oder einen aromatischen Rest, bedeutet, jeweils im etwa stöchiometrischen Mengenverhältnis, in Gegenwart von $AlCl_3$, gegebenenfalls in Gegenwart von inerten Schmelz- und/oder Lösungsmitteln, bei Temperaturen zwischen 40° und 150 °C einer Kondensationsreaktion unterwirft und das so erhaltene Reaktionsprodukt hydrolysiert.

Die Hydrolyse erfolgt zweckmässig durch Eingiessen der zunächst durch die Kondensationsreaktion erhaltenen Komplexverbindung in eine Mineralsäure/Wasser/Eis-Mischung. In manchen Fällen, insbesondere, wenn nach Entfernung aller Lösungsmittel der feste Rückstand pulverig zerfällt, ist auch die Hydrolyse durch Zugabe von verdünnten Mineralsäuren, wie Salzsäure, zu dem trockenen Produkt unter Kühlung möglich. Vorzugsweise wird die erfindungsgemässe Umsetzung wie oben beschrieben einstufig durchgeführt. Grundsätzlich lässt sich das Verfahren aber auch in der Weise 2-stufig ausführen, dass man in dem Reaktionsgemisch in der 1. Stufe in an sich bekannter Weise eine Ketocarbonsäure der Formel V

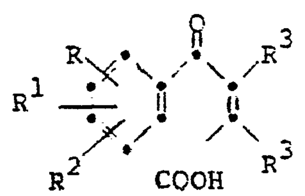

(V)

herstellt und dieselbe isoliert, und dass man in der 2. Stufe die Ketosäure der Formel V ebenfalls in Gegenwart von $AlCl_3$, gegebenenfalls in Gegenwart von inerten Schmelzund/oder Lösungsmitteln, aber bei Temperaturen zwischen 40° und 150 °C zur entsprechenden Indenoncarbonsäure der Formel I cyclisiert, welche durch Hydrolyse aus dem entstandenen Reaktionsgemisch freigesetzt wird.

Die Reaktionsbedingungen für die 2. Reaktionsstufe entsprechen also praktisch voll denjenigen des 1-Stufenverfahrens, nur mit dem Unterschied, dass in dieser 2. Reaktionsstufe anstelle der ursprünglichen Ausgangsprodukte der Formel II und III bzw. IV bereits das Zwischenprodukt der Formel V vorliegt.

Die Ausgangsprodukte der Formel II und III bzw. IV sind an sich bekannt und können nach üblichen Methoden hergestellt werden. Bevorzugt werden als Dihalogenmaleinsäureanhydrid der Formel III oder als Ester der Formel IV jeweils die Verbindungen eingesetzt, welche den Formel III und IV mit $R^3 = Cl$ entsprechen. Grundsätzlich können auch gemischte Halogenverbindungen, welche gleichzeitig Cl und Br enthalten, eingesetzt werden. Diese Produkte sind aber bekanntlich schwerer zugänglich als beispielsweise Dichlormaleinsäureanhydrid.

Als Ester der Formel IV kann eine Vielzahl von Verbindungen eingesetzt werden, da der Rest $R^4$ eine grosse Zahl von organischen Gruppen, welche sich von entsprechenden Alkoholen und Phenolen ableiten, darstellen kann.

Bevorzugt bedeutet $R^4$ einen aliphatischen Rest mit insgesamt 1 bis 4 C-Atomen, vorzugsweise $CH_3$.

In dem Reaktionsgemisch, das bei dem erfindungsgemässen Verfahren vorgelegt wird, sollte das $AlCl_3$ vorzugsweise in einer Menge von mindestens 1 Mol pro Mol des Dihalogenmaleinsäureanhydrid der Formel III oder des Esters der Formel IV vorliegen.

Wird erfindungsgemäss in Gegenwart von inerten Lösungsmitteln gearbeitet, was eine Vorzugsform des erfindungsgemässen Verfahrens darstellt, so sind solche Lösungsmittel bzw. Lösungsmittelgemische, welche einen Kochpunkt von mindestens 40 °C aufweisen, einzusetzen. Geeignete Lösungsmittel sind beispielsweise mehrfach halogenierte aliphatische und aromatische Kohlenwasserstoffe, wie o-, m- und p-Dichlorbenzol, Dichlormethan und 1,1,2,2-Tetrachloräthan. Ebenfalls geeignet sind Nitrobenzol und Dialkylamide niedermolekularer Carbonsäuren, wie Dimethylformamid oder Diäthylformamid. Bevorzugt angewandte Lösungsmittel sind 1,1,2,2-Tetrachloräthan und Nitrobenzol. Bei Nitrobenzol ist allerdings wegen der Explosionsgefahr die Reaktionstemperatur unter 90 °C zu halten. Auch die Dialkylamide niedermolekularer Carbonsäuren, vorzugsweise Dimethylformamid, sind recht wirkungsvoll als Lösungsmittel einsetzbar. Das Molverhältnis Dialkylamid zu $AlCl_3$ in dem Reaktionsgemisch sollte dabei mindestens 1 : 1 betragen.

Auch die Anwendung von inerten Schmelzmitteln stellt eine Vorzugsform des erfindungsgemässen Verfahrens dar. Als Schmelzmittel kommen anorganische Salze oder organische Schmelzmittel oder Gemische der anorganischen Salze mit den organischen Schmelzmitteln in Frage. Die Menge der angewendeten Schmelzmittel muss vorzugsweise so gross gewählt werden, dass im Gemisch mit dem $AlCl_3$ in der Reaktionsmischung ein Schmelzpunkt resultiert, welcher niedriger als die Reaktionstemperatur ist. Zweckmässig werden die eingesetzten Mengen so gewählt, dass im Gemisch mit dem $AlCl_3$ eine Schmelzpunktserniedrigung, beispielsweise aufgrund von Eutektikumbildung, resultiert.

Als organische Schmelzmittel werden erfindungsgemäss bevorzugt Dialkylamide niedermolekularer Carbonsäuren, wie z.B. Dimethyl- oder Diäthylformamid, eingesetzt. Die Menge sollte dabei vorzugsweise so gewählt werden, dass das Molverhältnis Dialkylamide zu $AlCl_3$ in den Grenzen 1 : 4 und 1 : 1 liegt.

Als anorganische Salze werden bevorzugt NaCl und/oder KCl, gegebenenfalls zusammen mit Dimethylformamid als organisches Schmelzmittel, eingesetzt. Weitere geeignete anorganische Schmelzmittel (insbesondere Mischungen) sind der Publikation von C.A. Thomas, « Anhydrous Aluminum Chloride In Organic Chemistry » (ACS Monogr. Ser.) (New York 1941), zu entnehmen.

Bei dem erfindungsgemässen Verfahren sind die jeweilige Reaktionstemperatur und -dauer wesentlich abhängig von der nucleophilen Reaktivität der verwendeten aromatischen Verbindung der Formel II. Dies bedingt wiederum die Wahl des Lösungs- bzw. Schmelzmittels. Im allgemeinen gelten folgende Regeln :

a) Beim Einsatz von reaktiven aromatischen Verbindungen der Formel II (welche 2 bis 3 $CH_3$-Gruppen als Substituenten am Sechsring enthalten) genügen Reaktionstemperaturen von 40° bis 80 °C, d.h. es sind sowohl organische Lösungs- oder Schmelzmittel als auch Schmelzmittel z.B. auf NaCl/KCl-Basis anwendbar.

b) Beim Einsatz von weniger reaktiven aromatischen Verbindungen der Formel II (welche H und Halogen am Sechsring enthalten) sind Reaktionstemperaturen von 80° bis 130 °C erforderlich ; hier sind auf jeden Fall Schmelzmittel anzuwenden, insbesondere anorganische Schmelzmittel auf Alkali- und/oder Erdalkalichloridbasis oder organische Schmelzmittel auf der Basis von Amiden niederer aliphatischer Carbonsäuren, wie Dimethylformamid.

Für eine wirtschaftliche Arbeitsweise sind bei der unter a) dargelegten Regel für die Reaktionsführung Reaktionszeiten erforderlich, welche im allgemeinen zwischen 0,5 und 12 Stunden liegen. Bei der unter b) beschriebenen Regel werden bevorzugt Reaktionszeiten zwischen 2 und 8 Stunden angewandt.

Weiterer Gegenstand der erfindung sind Verbindungen der der Formel VI

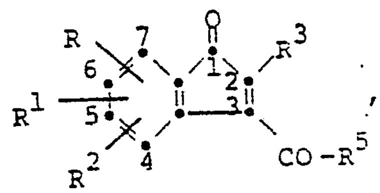 ,

(VI)

worin

$R^3$ für Cl oder Br, vorzugsweise für Cl, steht und

R, $R^1$ und $R^2$ gleich oder verschieden sind,

R einen n-Alkylrest mit 1 bis 4 C-Atomen, vorzugsweise $CH_3$, oder aber H, Cl, Br oder F,

$R^1$ einen n-Alkylrest mit 1 bis 4 C-Atomen, vorzugsweise $CH_3$, oder aber H,

$R^2$ H oder $CH_3$ oder aber

$R^1$ und $R^2$ zusammen die Gruppe $-CH_2CH_2CH_2-$ bedeuten, wobei im letzteren Fall die Bindung an den Sechsring über die C-Atome in 5- und 6-Position des Kerns erfolgt, worin $R^5$ einen der Reste

$-OH$,

$-O-(CH_2)_m-OH$,

$-O-(CH_2-CH_2-O)_n-CH_2-CH_2-OH$,

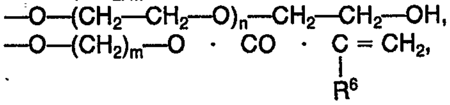

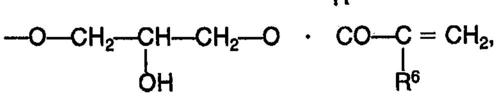

$-O-CH_2-CH_2-O-CH=CH_2$ und

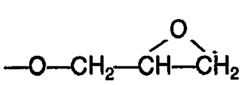

bedeutet,

wobei m eine Zahl von 2 bis 6, n eine Zahl von 1 bis 10, und $R^6$H oder $-CH_3$ darstellen, mit der Massgabe, dass in dem Fall, in dem $R^5$—OH bedeutet, mindestens ein Wasserstoffatom in 4- bis 7-Stellung des Sechsringes substituiert ist.

Bevorzugte Verbindungen der Formel VI sind diejenigen, welche der Formel VI mit der Bedeutung von —OH für $R^5$ entsprechen, d.h. die freien Halogen-indenoncarbonsäuren, insbesondere die Chlorprodukte. Entsprechende bevorzugte Ester sind solche der Formeln VII und VIII

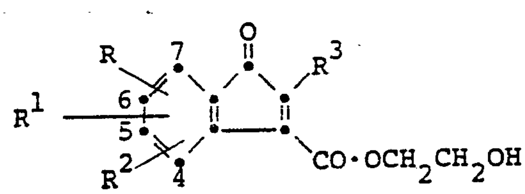

(VII)

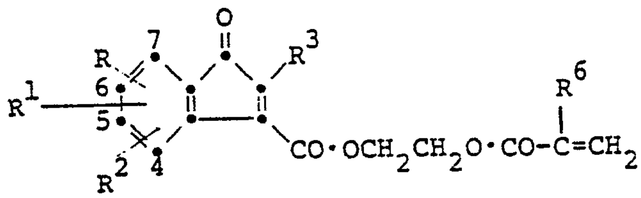

(VIII)

wobei die angegebenen Reste R bis $R^6$ die vorher angegebene Bedeutung haben. Bevorzugt bedeutet $R^3$ in den Formeln VII und VIII jeweils Chlor.

Besonders bevorzugt sind die Verbindungen der Formeln XVII, XVIII und XIX :

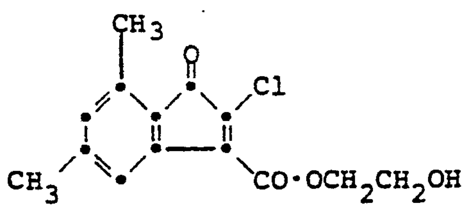

(XVII)

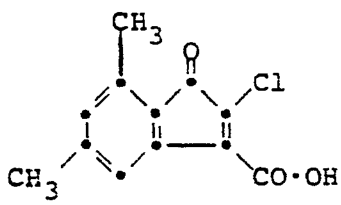

(XVIII)

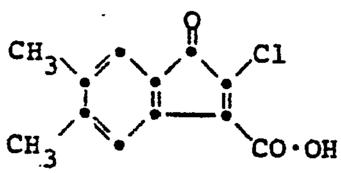

(XIX)

Die Herstellung der Ester der Halogenindenoncarbonsäuren der Formel VI erfolgt nach an sich bekannten Verfahren. Im einzelnen ist dazu genauer das Folgende auszuführen.

Die Herstellung der Ester der Formel VI, in der $R^5$ den Rest $-O-(CH_2)_m-OH$ bedeutet, erfolgt durch Veresterung der entsprechenden Halogen-indenoncarbonsäure mit dem jeweiligen Alkylenglykol in Gegenwart von inerten organischen Lösungsmitteln (z.B. Aethylenglykol-dimethyläther) und Veresterungskatalysatoren (z.B. Schwefelsäure).

Die Herstellung der Ester der Formel VI, in der $R^5$ den Rest $-O-(CH_2-CH_2 \cdot O)_n-CH_2-CH_2-OH$ bedeutet, erfolgt durch Addition von n Molekülen Aethylenoxid an die jeweilige Halogen-indenoncarbonsäure.

Die Ester der Formel VI, in der $R^5$ den Rest $-O-(CH_2)_m-O \cdot CO-\underset{R^6}{C}=CH_2$ bedeutet, werden durch Veresterung des jeweiligen Hydroxy-alkylesters (d.h. $R^5$ bedeutet den Rest $-O-(CH_2)_m-OH$) mit Acrylsäure oder Methacrylsäure hergestellt.

Die Ester der Formel VI, in der $R^5$ den Rest $-O-CH_2-\underset{OH}{CH}-CH_2-O \cdot CO-\underset{R^6}{C}=CH_2$ bedeutet, werden entweder durch Umsetzung von Acrylsäure- oder Methacrylsäureglycidylester mit der jeweiligen Halogen-indenoncarbonsäure oder aber durch Umsetzung von Acryl- oder Methacylsäure mit dem jeweiligen Halogen-indenoncarbonsäureester der Formel VI, in der $R^5$ den Rest

$-O-CH_2-\overset{O}{\overbrace{CH-CH_2}}$ bedeutet, hergestellt.

Die Herstellung des zuletzt genannten Produktes der Formel VI erfolgt durch Umsetzen von Epichlorhydrin mit der jeweiligen Halogenindenoncarbonsäure.

Die Ester der Formel VI, in der $R^5$ den Rest $—O—CH_2—CH_2—O—CH = CH_2$ bedeutet, werden durch Kondensation von α-Chloro-äthylvinyläther und der jeweiligen Halogenindenoncarbonsäure hergestellt.

Bei allen Verfahren zur Herstellung der Halogenindenoncarbonsäureester der Formel VI wird vorzugsweise in Gegenwart von inerten organischen Lösungsmitteln gearbeitet.

Die neuen, verbesserten, unter der Einwirkung von Licht vernetzbaren Polymeren, welche sich unter Verwendung von Verbindungen der Formel VI als Ausgangssubstanzen herstellen lassen, enthalten seitenständige Gruppen der Formel IX

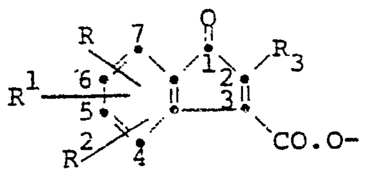

(IX)

wobei die Reste R bis $R^3$ dieselbe Bedeutung wie in Formel I haben. Der Anteil an Gruppen der Formel IX beträgt dabei 5 bis 100, bevorzugt 20 bis 100 %, bezogen auf die Anzahl der wiederkehrenden Strukturelemente des jeweiligen Polymeren.

Bei diesen lichtempfindlichen Polymeren handelt es sich z.B. um solche aus der Gruppe der Phenol-Formaldehyd-Harze, der Novolake, der Phenoxyharze und solche, welche durch Homo- oder copolymerisation von C = C-Doppelbindungen enthaltenden Monomeren erhalten werden.

Die Polymere lassen sich nach an sich bekannten Synthesemethoden zur Herstellung von Makromolekülen mit photoaktiven seitenständigen Gruppen herstellen. Grundsätzlich kommen folgende Wege in Betracht :

1. Einbau der Gruppe Formel IX in eine bestehende Polymerkette ;

2. Aufbau der Polymerkette aus Monomeren, welche die lichtempfindliche Gruppe der Formel IX bereits enthalten, wobei dieser Kettenaufbau vorzugsweise durch eine Polymerisation über C = C-Doppelbindungen erfolgt.

Bei dem 1. Herstellungsweg wird stets in Gegenwart von inerten Lösungsmitteln gearbeitet. Bei der 2. Methode wird entweder in Block oder in Gegenwart von inerten Lösungsmitteln polymerisiert. Da beide Verfahren nach an sich bekannten Grundmethoden durchgeführt werden, erübrigen sich hier weitere Angaben über die gegebenenfalls zu verwendenden Lösungsmittel, Katalysatoren und Temperaturen. Derartige Grundmethoden sind im übrigen ausführlich in dem US-Patent 4079 041 beschrieben.

Teilweise können mit den Methoden 1 und 2 gleiche Produkte erhalten werden, so dass sich wahlweise die Methode 1 oder 2 anwenden lässt. Werden die Gruppen der Formel IX in eine bereits vorhandene Polymerkette eingebaut, so erfolgt dieser Einbau beispielsweise durch eine Additionsreaktion unter gleichzeitiger Oeffnung eines Ringsystems, z.B. einer Dicarbonsäureanhydridgruppe oder einer Epoxidgruppe.

Eine mögliche Form dieser Polymeren stellt ein solches Homo- oder Copolymerisat von reaktiven Doppelbindungen enthaltenden Monomeren dar, welches Durchschnittsmolekulargewichte zwischen 1 000 und 1 000 000 aufweist, und welches die Gruppen der Formel IX in Molekülkettengliedern der Formeln a bis e

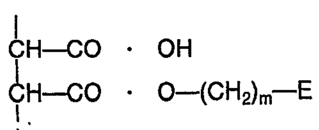

(a)

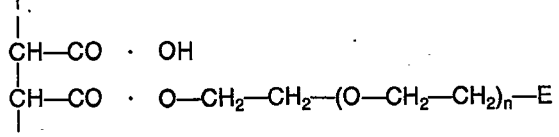

(b)

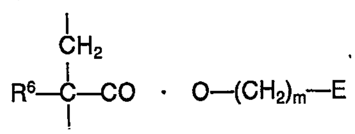

(c)

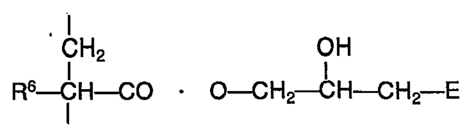

(d)

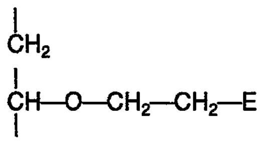

(e)

enthält, in denen der Rest E der Formel IX entspricht, m eine Zahl von 2 bis 6, n eine Zahl von 1 bis 10 und $R^6$H oder $—CH_3$ darstellen.

Ein solches Polymer kann ausser einem oder mehreren der Strukturelemente der Formel a bis e gleichzeitig Strukturelemente der Formel f

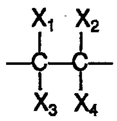

(f)

enthalten, worin

$X_1$ und $X_3$ je Wasserstoff, $X_2$ Wasserstoff, Chlor oder Methyl, $X_4$ Wasserstoff, Methyl, Chlor, —CN, —COOH, Phenyl, Methylphenyl, Methoxyphenyl, Cyclohexyl—, —COO—Alkyl mit 1-2 C-Atomen im Alkylteil, —COO—Phenyl, 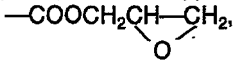,

—COO—Alkyl—OH mit 1-3 C-Atomen im Alkylteil, —OCO—Alkyl mit 1-4 C-Atomen im Alkyl, —OCO—Phenyl, —CO—Alkyl mit 1-3 C-Atomen in Alkyl, Alkoxy mit 1-6 C-Atomen oder Phenoxy oder $X_1$ und $X_2$ je Wasserstoff und $X_3$ und $X_4$ zusammen die Gruppierung 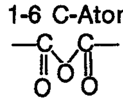 oder je —COOH oder —COO-Alkyl

1-6 C-Atomen im Alkyl bedeuten.

Bevorzugt werden bei diesen speziellen Polymeren, solche, welche Strukturelemente der Formel f enthalten, worin $X_1$ und $X_3$ je Wasserstoff, $X_2$ Wasserstoff oder Methyl und $X_4$ $—OCOCH_3—$ —COOH oder —COOAlkyl mit 1-8 C-Atomen im Alkyl bedeuten oder worin $X_1$, $X_2$ und $X_3$ je Wasserstoff und $X_4$ —CN, Chlor oder Phenyl bedeuten.

Ein weiteres mögliches Polymer, welches sich bei Ausgang von den erfindungsgemässen Verbindungen der Formel VI herstellen lassen, ist ein solches auf der Ausgangsbasis Novolak, welches Strukturelemente der Formel g

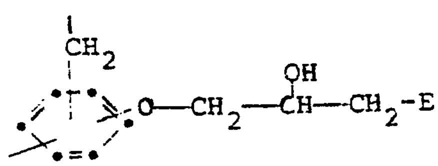

(g)

in der der Rest E der Formel IX entspricht, aufweist und dessen Durchschnittsmolekülargewicht zwischen 1 000 und 100 000 liegt.

Ein Teil der lichtempfindlichen Polymeren mit Gruppen der Formel IX kann z.B. dadurch hergestellt werden, dass man ein freie —OH—Gruppen enthaltendes Polymer, vorzugsweise ein Kunstharz vom Typ Novolak, Phenoxyharz oder Phenol-Formaldehyd-Harz, mit einer Verbindung der Formel XI

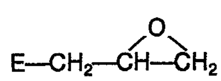

(XI)

in der E der Formel IX entspricht, umsetzt.

Man Kann aber auch in der Weise vorgehen, dass man ein anstelle der Freien —OH—Gruppen Glycidylgruppen enthaltendes Kunstharz, vorzugsweise vom Typ Novolak, Phenoxyharz oder Phenol-Formaldehyd-Harz, mit einer Halogenindenoncarbonsäuren der Formel E-H, in der der Rest E die Gruppe der Formel IX darstellt, umsetzt.

Nach beiden Arbeitsweisen lassen sich auch die bevorzugten Polymeren, welche Strukturelemente der Formel g aufweisen, darstellen.

Die Polymere, welche die Gruppen der Formel IX in Molekülkettengliedern der Formel a und b enthalten, kann man in der Weise herstellen, dass man ein Homopolymer oder ein Mischpolymer von Maleinsäureanhydrid, welche ein gegebenenfalls substituiertes Maleinsäureanhydrid einpolymerisiert enthalten, wobei die Anhydridgruppe ganz oder teilweise erhalten ist, mit einer der Verbindungen der Formeln XII und XIII

$E-(CH_2)_m-OH$ (XII)

$E-(CH_2-CH_2-O)_n-CH_2-CH_2-OH$ (XIII),

in denen m eine Zahl von 2 bis 6 und n eine Zahl von 1 bis 10 bedeuten, und in denen E der Formel IX entspricht, umsetzt.

Bei der Herstellung dieser zuletzt beschriebenen oder ähnlicher Polymeren kann man aber auch in der Weise vorgehen, dass man in erster Reaktion Maleinsäureanhydrid, welches gegebenenfalls substituiert ist, mit einer der Verbindungen der Formeln (XII) oder (XIII) umsetzt, und in einer 2. Reaktion das jeweils erhaltene ungesättigte Monomer, gegebenenfalls zusammen mit anderen mindestens eine C = C-Doppelbindung aufweisenden Comonomeren polymerisiert.

Als Ausganssubstanzen für diese Polymere wird vorzugsweise Maleinsäureanhydrid verwendet. Grundsätzlich lassen sich aber auch entsprechend Polymere herstellen, welche sich von substituierten Maleinsäureanhydriden ableiten. Dabei handelt es sich insbesondere um methylsubstituierte Maleinsäureanhydride. Geeignete Comonomere für Maleinsäureanhydrid sind beispielsweise Styrol, Vinyläther, Aethylen und Propylen.

Die Polymeren, welche die Gruppen der Formel IX in Molekülkettengliedern der Formeln c bis e enthalten, lassen sich in der Weise herstellen, dass man eine oder mehrere der Verbindungen der Formeln XIV bis XVI

$$CH_2=\underset{\displaystyle R^6}{\underset{|}{C}}-CO \cdot O-(CH_2)_m-E \qquad (XIV)$$

$$CH_2=\underset{\displaystyle R^6}{\underset{|}{C}}-CO \cdot O-CH_2-\underset{\displaystyle OH}{\underset{|}{CH}}-CH_2-E \qquad (XV)$$

$$CH_2=CH-O-CH_2-CH_2-E \qquad (XVI)$$

in denen $R^6$ H oder $CH_3$ darstellt und m und E dieselbe Bedeutung wie in Formel a haben, gegebenenfalls zusammen mit weiteren Comonomeren, polymerisiert.

Geeignete Comonomere für die Monomeren der Formeln XIV bis XVI sind beispielsweise Acrylate und Methacrylate, welche nicht den Rest E enthalten, Styrol Vinylverbindungen, wie Vinyläther, Vinylchlorid und Vinylacetat, Vinylidenchlorid.

Die Polymeren mit Molekülkettengliedern der Formel c lassen sich aber auch in der Weise herstellen, dass man ein Polyacrylsäure- oder ein Polymethacrylsäurechlorid oder ein entsprechendes Mischpolymer jeweils mit einem Ester der Formel $E-(CH_2)_m-OH$, in der E die Gruppe der Formel IX darstellt und m eine Zahl von 2 bis 6 bedeutet, kondensiert. Unter Mischpolymeren sind hier solche zu verstehen, welche dieselben Comonomeren wie für die Monomeren der Formeln XIV bis XVI enthalten.

Die Polymeren mit Molekülkettengliedern der Formel d können auch durch Umsetzen von Polyacrylsäureglydidylestern oder von Polymethacrylsäureglycidylestern oder von entsprechenden Mischpolymeren mit glycidylgruppenfreien Monomeren mit Halogenindenoncarbonsäuren der Formel E-H hergestellt werden.

Nach der jeweiligen beendeten Umsetzung kann das Polymer durch Eingiessen in geeignete organische Lösungsmittel, z.B. aliphatische Kohlenwasserstoffe, Alkohole oder Dialkyläther, wie n-Pentan, n-Hexan, Methanol, Aethanol und Diäthyläther, ausgefällt werden. Es liegt dann als orangegefärbtes Produkt vor.

Die durch Licht vernetzbaren Polymere eignen sich beispielsweise zur Herstellung von Druckplatten für das Offsetdruckverfahren, zur Herstellung von Photooffset-lacken, für die unkonventionelle Photographie zum Abfärben von nach dem Belichten und Entwickeln sichtbaren Polymerbildern mit geeigneten Farbstoffen, wie öllösliche Farbstoffe oder, wenn das Polymere saure Gruppen, wie Carbonsäure- oder Sulfonsäuregruppen aufweist, kationische Farbstoffe. Die Polymere finden insbesondere Anwendung als sogenannte Photoresists zur Herstellung von gedruckten Schaltungen nach an sich bekannten Methoden.

## Herstellungsbeispiele

### Beispiel 1

In einem mit HCl-Ableitung versehenen Rührkolben wird eine Mischung bestehend aus 88 g pulverisiertem wasserfreien $AlCl_3$, 16 g NaCl, 5,4g KCl und 16,7 g (0,1 Mol) Dichlormaleinsäureanhydrid vorgelegt und für kurze Zeit (bis geschmolzen) auf 90-100 °C aufgeheizt. Bei 70-75 °C fügt man im Verlaufe von 45 Minuten 10,6 g (0,1 Mol) m-Xylol zu. Nach weiteren 30 Minuten Rühren bei 75-80 °C wird

die Schmelze in ein Gemisch aus 25 ml konz. Salzsäure, Wasser und Eis eingetragen (Endvolumen ca. 1 Liter) und das Produkt abfiltriert. Nach dem Waschen mit Wasser und Trocknung im Vakuum bei 60 °C erhält man 23,0 g (97 % d.Th.) gelborange 1-Oxo-2-chlor-5,7-dimethyl-inden-carbonsäure-(3). Gemäss Dünnschichtchromatogramm enthält das Produkt nur geringfügige Verunreinigungen und kann daher direkt für weitere Umsetzungen verwendet werden. Das aus Essigsäureaethylester umkristallisierte Produkt schmilzt bei 255-256 °C.

$^1$H-NMR-Spektrum (100 mHz, /-Werte in ppm, Lösung in $(CD_3)_2SO$) : 2,27 (S, 3H, $-CH_3$) ; 2,38 (S, 3H, $-CH_3$) ; 6,8 und 7,2 (2H, aromat.) ; ca. 13,5 (breites Signal, 1H, $D_2O$ austauschbar).

Chemische Analyse und $^1$H-NMR-Spektrum entsprechen der Formel

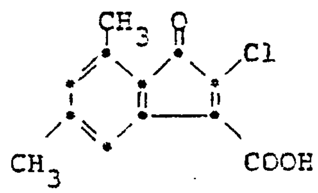

Auf analoge Weise wurden die in der folgenden Tabelle aufgeführten Indenone hergestellt.

(Siehe die Tabelle, Seite 11)

| Beispiel Nr. | Verbindung der Formel b | 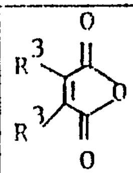 | Eintragen der Verb. der Formel b in die Schmelzen | | Reaktion nach dem Eintragen von Verb. der Formel b | | Verbindung der Formel a | Ausbeute % d.Th. | umkristallisiert aus | Smp. |
|---|---|---|---|---|---|---|---|---|---|---|
| | | | Dauer h | Temper. °C | Dauer h | Temper. °C | | | | |
| 2 | m-Xylol | $R^3$=Br | 1 | 70-75 | 0,5 | 75-80 | 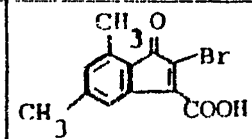 | 79 | Essigsäure-äthylester | 257 |
| 3 | Benzol | $R^3$=Cl | 0,75 | 70-75 | 2 | 80-85 | 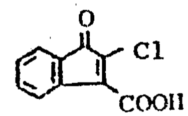 | 90 | Wasser + Aethanol (1:1) | 228 |
| 4 | Toluol | $R^3$=Cl | 0,75 | 70-75 | 1,5 | 80-85 | 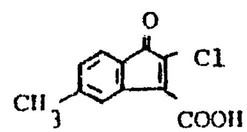 | 91 | Essigsäure-äthylester | 239 |
| 5 | p-Xylol | $R^3$=Cl | 0,75 | 70-75 | 2 | 80-85 | 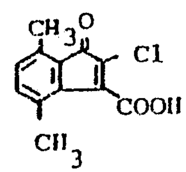 | 88 | Chlorbenzol | 170 |
| 6 | 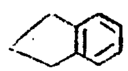 | $R^3$=Cl | 1 | 70-75 | 1 | 75-80 | 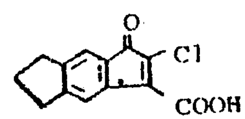 | 95 | Toluol | 239 |

| Beispiel Nr. | Verbindung der Formel b | 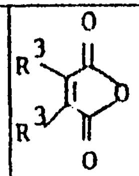 | Eintragen der Verb. der Formel b in die Schmelzen | | Reaktion nach dem Eintragen von Verb. der Formel b | | Verbindung der Formel a | Ausbeute % der Theorie | umkristallisiert aus | Smp. |
|---|---|---|---|---|---|---|---|---|---|---|
| | | | Dauer h | Temper. °C | Dauer h | Temper. °C | | | | |
| 7 | Monochlorbenzol | $R^3$=Cl | 1 | 80-85 | 2,5 | 95-100 | 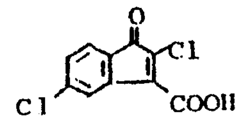 | 55 | Essigsäureäthylester | 258 |
| 8 | 1,2,3-Trimethylbenzol | $R^3$=Cl | 1 | 75-80 | 0,5 | 75-80 | 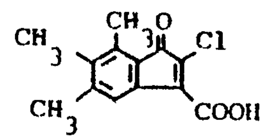 | 95 | Eisessig | 225 |
| 9 | 1,2,4-Trimethylbenzol | $R^3$=Cl | 1 | 75-80 | 0,5 | 75-80 | 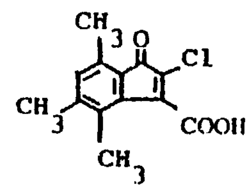 | 97 | Toluol | 193 |
| 10 | n-Butylbenzol | $R^3$=Cl | 0,75 | 75-80 | 1 | 80-85 | 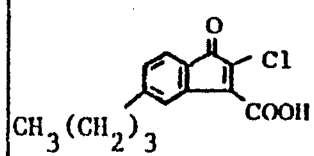 | 98 | Cyclohexan | 157 |
| 11 | Aethylbenzol | $R^3$=Cl | 0,5 | 95-100 | 0,5 | 95-100 | 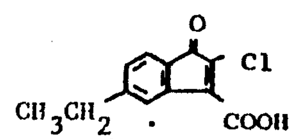 | 99 | Cyclohexan | 150 |

Beispiel 12

In einem Rührkolben mit HCl-Ableitung wird eine Mischung, bestehend aus 53 g pulverisiertem wasserfreien $AlCl_3$ und 16,7 g (0,1 Mol) Dichlormaleinsäureanhydrid in 80 ml 1,2-Dichloraethan vorgelegt. Dann fügt man bei 20-30 °C innerhalb von etwa 30 Minuten 7,8 g (0,1 Mol) Benzol zu und rührt bei der angegebenen Temperatur, bis eine Probe im Dünnschichtchromatogramm vollständige Umsetzung anzeigt. Die Reaktionsmass wird sodann in ein Gemisch aus 25 ml konz. Salzsäure, Wasser und Eis eingetragen (Endvolumen ca 600 ml) und die Dichloraethanlösung im Vakuum zur Trockene eingedampft. Man erhält 23,5 g β-Benzoyl-dichloracrylsäure, welche unter analogen Bedingungen wie bei der Herstellung der Chlorindenoncarbonsäure nach dem 1-Stufenverfahren gemäss Beispiel 3, d.h. in Gegenwart desselben Schmelzmittelgemisches und unter Anwendung der gleichen Temperaturen, cyclisiert wird.

Ausbeute 17,0 g, entsprechend 82 % d.Th. Smp.: 228 °C.

Auf analoge Weise lassen sich nach diesem zweistufigen Verfahren die anderen Halogen-indenoncarbonsäuren der Beispiele 1 und 2, sowie 4 bis 9 darstellen.

Die gleichen Endprodukte werden erhalten, wenn in obigen Beispielen anstelle von Dichlor- bzw. Dibrom-maleinsäureanhydrid ein Säurechlorid der Formel

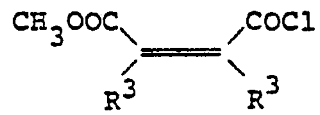

$R^3$ = Cl oder Br

in äquimolekularer Menge verwendet wird.

Beispiel 13

Man setzt gemäss Beispiel 12, 7,8 g (0,1 Mol) Benzol mit Dichlormaleinsäureanhydrid in Gegenwart von $AlCl_3$ um und filtriert die erhaltene braune Reaktionslösung (ohne Zersetzung mit verdünnter Salzsäure) von etwas ungelöstem $AlCl_3$ ab. Das Filtrat wird im Rotationsverdampfer im Vakuum zur Trockene eingedampft. Den Rückstand erhitzt man während 30 Min. auf 150 °C, wobei er pulverig zerfällt. Nach üblicher Aufarbeitung durch Eintragen in verdünnte Salzsäure, Isolierung und Trocknung erhält man ein Rohprodukt, welches durch Umkristallisation aus Chlorbenzol von schwerlöslichem dunklem Nebenprodukt befreit wird. Man erhält 6,2 g entsprechend 30 % d.Th. 1-Oxo-2-chlor-inden-carbonsäure-(3).

Beispiel 14

In einem Rührkolben mit HCl-Ableitung werden 14,6 g (0,2 Mol) Dimethylformamid vorgelegt und langsam 54 g (0,4 Mol) pulverisiertes wasserfreies $AlCl_3$ und 16,7 (0,1 Mol) Dichlormaleinsäureanhydrid eingetragen, wobei durch Kühlung die Temperatur auf unter ca. 80 °C gehalten wird. Bei 70-75 °C fügt man im Verlaufe von 45 Minuten 10,6 g (0,1 Mol) m-Xylol zu und rührt die dunkle Schmelze während 1,5 Stunden bei 75-80 °C. Die weitere Aufarbeitung geschieht wie in Beispiel 1 beschrieben. Man erhält 22,9 g, entsprechend 97 % d.Th. an chromatographisch annähernd reiner 1-Oxo-2-chlor-5,7-dimethyl-inden-carbonsäure-(3).

Auf analoge Weise lassen sich die in den Beispielen 2 bis 9 beschriebenen Halogenindenoncarbonsäuren darstellen. Es werden hierbei die in den betreffenden Beispielen angegebenen Reaktionstemperaturen verwendet. Die Reaktionsduaer (nach dem Eintragen von Verbindung der Formel (II)) wird hierbei auf das Dreifache der angegebenen Zeitdauer verlängert.

Beispiel 15

In einem Rührkolben mit HCl-Ableitung werden 54 g pulverisiertes wasserfreies $AlCl_3$ und 10,6 g (0,1 Mol) m-Xylol in 70 ml 1,1,2,2-Tetrachloraethan vorgelegt. Dann fügt man innerhalb von etwa 30 Minuten 17,8 g (0,1 Mol) Dichlormaleinsäure-anhydrid zu und rührt bei 20-30 °C während 2 Stunden. Hierauf hält man die Mischung während 10 Stunden auf einer Temperatur von 55-60 °C und giesst in Eiswasser, welches 25 ml konz. Salzsäure enthält. Die Tetrachloraethanlösung wird abgetrennt, mit ca. 250 ml Wasser versetzt und im Rotationsverdampfer unter Vakuum das Lösungsmittel abdestilliert. Das Produkt wird abfiltriert, mit etwas Wasser gewaschen und in 800 ml Wasser unter Zusatz der notwendigen Menge Natriumcarbonat bei Raumtemperatur gelöst. Nach Abtrennung einer unlöslichen Verunreinigung durch Filtration wird das Filtrat mit überschüssiger Salzsäure versetzt und das ausgefällene Produkt auf übliche Weise isoliert. Man erhält 20,5 g (87 % d.Th.) 1-Oxo-2-chlor-5,7-dimethyl-indencarbonsäure-(3), deren Eigenschaften den im Beispiel 1 beschriebenen entsprechen.

Beispiele 16 bis 18

Auf analoge Weise werden dargestellt: Unter Verwendung von o-Xylol die 1-Oxo-2-chlor-5,6-dimethylinden-carbonsäure-(3), 98 % d.Th., Smp. 229° (umkristalliert aus Eisessig). Aus p-Xylol wird das Beispiel 5 entsprechende Produkt erhalten und aus 1,2,4-Trimethylbenzol erhält man die 1-Oxo-2-chlor-4,5,7-trimethylindencarbonsäure-(3) vom Smp. 194° (umkristalliert aus Toluol).

Beispiel 19

71 g (0,3 Mol) 1-Oxo-2-chlor-5,7-dimethylinden-carbonsäure-(3) werden in 400 ml Aethylenglykol-dimethyläther und 250 ml Aethylenglykol suspendiert. Unter Rühren fügt man 70 ml konzentrierte Schwefelsäure zu und hält die Mischung während 48 Stunden auf einer Temperatur von 55-60°. Die Reaktionsmasse wird in Eiswasser eingetragen und abfiltriert. Der Nutschenrückstand wird in 2 Liter Wasser angeschlämmt, mit Natriumcarbonat auf pH 8 gestellt, filtriert und mit 500 ml Wasser gewaschen. Man erhält nach Trocknung 42 g des Esters der Formel

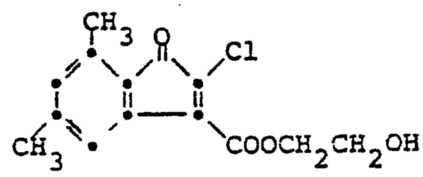

Schmelzpunkt 112° (umkristalliert aus Toluol).

Aus dem sodaalkalischen Filtrat können durch Ausfällung mit Salzsäure 22 g nicht veresterte Carbonsäure zurückgewonnen und wieder eingesetzt werden.

Beispiel 20

2-Chlor-indenoncarbonsäurester der Formel

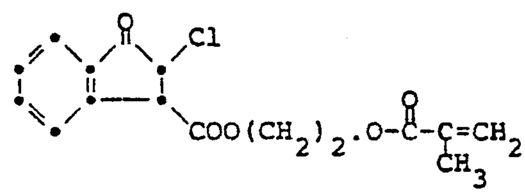

2,0 g (0,03 M) Chlorindenoncarbonsäure-2-hydroxy-äthylester werden in 20 ml trockenem Methylenchlorid gelöst. Zu dieser Lösung werden 3 g (0,03 M) Natriumcarbonat zugefügt. Zu diesem Gemisch wird eine Lösung von 3,5 g (0,033 M) Methacrylsäurechlorid in 5 ml trockenem Methylenchlorid zugetropft.

Nach erfolgter Reaktion wird die Lösung durch Filtration von gebildetem Natriumchlorid und Soda abgetrennt.

Das Filtrat wird neutral gewaschen und anschliessend am Rotationsverdampfer zur Trockene eingedampft.

Ausbeute an Ester: 84 % d.Th.

Fp: 98-100 °C

NMR($CDCl_3$): 2,0 ppm [3H], 2,6 ppm [4H]
5,6 bzw. 6,2 ppm [2H], 7,1-7,8 ppm [4H]

EA:

| | | | |
|---|---|---|---|
| Berechnet: | 59,92 % C; | 4,09 % H; | 11,05 % Cl |
| Gefunden: | 59,86 % C; | 4,03 % H; | 11,27 % Cl |

$C_{16}H_{13}O_5Cl$
(320,73)

Anwendungsbeispiele

Beispiel I

2,5 g eines copolymeren von Methylmethacrylat und Glycidyl-Methacrylat [(Mol-Verhältnis 1 : 1), Mw 60 000] werden zusammen mit 1,8 g (8,63 mM) Chlorindenoncarbonsäure und 0,01 g Tetramethylammoniumchlorid in 20 g Cyclohexanon gelöst. Diese Lösung wird während ca. 2 Stunden auf 120 °C erhitzt.

Nach dem Abkühlen auf Raumtemperatur wird die Lösung filtriert. Das Filtrat kann direkt als Beschichtungslösung zur Herstellung von Photolackschichten verwendet werden.

Das erhaltene Polymer weist neben Strukturelementen der Formel

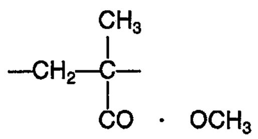

Strukturelemente der Formel

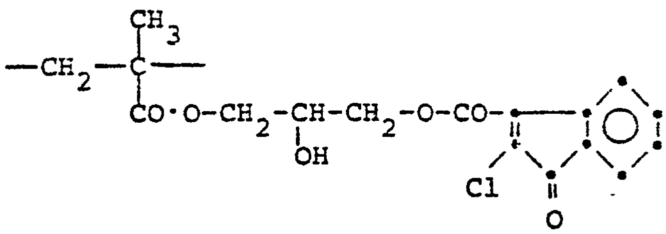

auf. Es ist wie alle in den folgenden Beispielen beschriebenen Polymeren orange gefärbt.

## Beispiel II

10 g eines Copolymeren von Methylvinyläther und Maleïnsäureanhydrid (Mol-Verhältnis 1 : 1 alternierend, Mw 740 000), 8 g (0,03 M) Chlorindenoncarbonsäure-2-hydroxyäthylester und 0,15 ml conc. Schwefelsäure werden in 120 ml trockenem Tetrahydrofuran gelöst. Die Lösung wird während 24 Stunden unter Stickstoff am Rückfluss gekocht. Anschliessend wird auf Raumtemperatur abgekühlt, die Lösung filtriert. Das Filtrat kann direkt zu Beschichtungszwecken verwendet werden.

Zur Bestimmung der Zusammensetzung wird eine kleine Probe des Filtrates in Aether ausgefällt.

Elementaranalyse :

| | | | |
|---|---|---|---|
| Berechnet : | 55,27 % C ; | 4,46 % H ; | 6,28 % Cl |
| Gefunden : | 55,85 % C ; | 4,59 % H ; | 5,56 % Cl |

## Beispiel III

2,66 g eines Copolymeren von Aethylen und Maleinsäureanhydrid (1 : 1 alternierend, Mw 100 000) und 5,34 g (0,020 Mol) Chlorindenoncarbonsäure-2-hydroxyäthylester werden in 40 ml trockenem N-Methylpyrrolidon gelöst. Diese Lösung wird während 48 Stunden bei 100 °C gekocht. Nach Abkühlen auf Raumtemperatur wird filtriert. Das Filtrat kann direkt zu Beschichtungszwecken eingesetz werden. Eine Probe wird in Aether gefällt, und die Grenzviskositätszahl des Produkts bestimmt. ($\eta$ intr.) (DMF, 20 °C) : 0,3 dl/g.

## Beispiel IV

Es wird analog Beispiel I gearbeitet. Als Ausgangspolymer wird Gantrez 119 AN[R] verwendet. Es handelt sich um ein Handelprodukt der Firma GAF Corp., USA, und zwar um ein Copolymer von Vinylmethyläther und Maleinsäureanhydrid mit einem Mw von ungefähr 740 000. Als Chlorindenonsäurederivat wird der Chlor-indenoncarbonsäure-2-hydroxyäthylester eingesetzt. Das Gewichtsverhältnis dieser Reaktionspartner beträgt 2 : 5.

Es resultiert ein lichtempfindliches Polymer mit einer Grenzviskositätszahl ($\eta$ intr.) von 0,25 dl/g (DMF ; 20 °C).

## Beispiel V

Es wird analog Beispiel IV verfahren, nur mit dem Unterschied, dass 2-Chlor-5,7-dimethyl-indenoncarbonsäure-2-hydroxy-äthylester anstelle von Chlor-indenoncarbonsäure-2-hydroxyäthylester eingesetzt wird und dass das Gewichtsverhältnis der Reaktionspartner 2 : 2,32 anstelle von 2 : 5 beträgt. Es resultiert ein durch Licht vernetzbares Polymer mit einer $\eta$ intr. von 0,22 dl/g (DMF : 20 °C).

## Beispiel VI

10,45 g Polymethacrylsäurechlorid (Mw 40 000), gelöst in 10 ml trockenem Toluol, werden zusammen mit 12,9 g (0,05 M) Chlorindenoncarbonsäure-2-hydroxyäthylester in 80 ml Chlorbenzol gelöst. Zu dieser

Lösung werden 10 g fein pulveriertes 3-Å-Molekularsieb beigefügt. Diese Lösung wird am Rückfluss während ca. 2 Stunden gekocht. Nach dem Abkühlen auf Raumtemperatur wird die Reaktionslösung filtriert und das Filtrat anschliessend in 2l Methanol gefällt. Das resultierende, helle Pulver weist nach dem Trocknen eine Grenzviskositätszahl, η intr. (DMF, 20 °C) von 0,1 dg/g auf.

Beispiel VII

Die gemäss Beispiel I hergestellte Beschichtungslösung wird zur Beschichtung von kupferkaschierten Epoxilaminaten verwendet:

Mittels einer Beschichtungszentrifuge werden kupferkaschierte Epoxiplatten mit der oben erwähnten Polymerlösung beschichtet. Nach dem Trocknen resultiert ein ca 5 μm dicker Polymerfilm auf der Kupferoberfläche. Die so beschichtete trockene Platte wird durch ein Strichnegativ mit UV-Licht (Wellenlänge über 320 nm) Belichtungsquelle: Quecksilberhochdrucklampe 400 W, Abstand 50 cm/während 3' belichtet. Nach dem Belichten ist das entstandene Bild bereits sichtbar, durch Entwickeln in Cyclohexanon werden die nicht belichteten Anteile herausgelöst. Das blanke Kupfer wird durch Aetzen mit Eisen(III)-chlorid herausgelöst, wobei ein dem Strichnegativ entsprechendes Kupferbild erhalten wird.

Beispiel VIII

Die gemäss Beispiel IV hergestellte Beschichtungslösung wird zur Beschichtung von Aluminiumfilmen verwendet:

Mittels einer Beschichtungszentrifuge wird die Aluminiumfolie mit der oben erwähnten. Polymerlösung beschichtet und anschliessend getrocknet. Nach dem Tocknen resultiert ein ca 5 μm dicker Polymerfilm auf dem Aluminiumträger. Die so beschichtete Platte wird durch ein Strichnegativ mit UV-Licht (Wellenlänge über 320 nm, Belichtungsquelle: Quecksilberhochdrucklampe 400 W, Abstand 50 cm) während 1' belichtet. Nach dem Belichten ist das entstandene Bild bereits sichtbar, durch Entwickeln in 5 %-iger Natriumbicarbonatlösung werden die nicht belichteten Anteile heraus gelöst. Das resultierende Reliefbild kann mittels kationischer Farbstoffe wie z.B. Maxilonrot intensiver gemacht werden. Es entsteht dabei die gefärbte Abbildung der Negativvorlage.

**Ansprüche**

1. Verfahren zur Herstellung von Indenoncarbonsäuren der Formel I

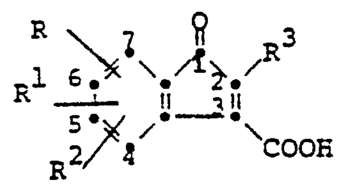

(I)

worin

$R^3$ für Cl oder Br, vorzugsweise für Cl, steht und

R, $R^1$ und $R^2$ gleich oder verschieden sind,

R einen n-Alkylrest mit 1 bis 4 C-Atomen, vorzugsweise $CH_3$, oder aber H, Cl, Br oder F,

$R^1$ einen n-Alkylrest mit 1 bis 4 C-Atomen, vorzugsweise $CH_3$, oder aber H,

$R^2$ H oder $CH_3$ oder aber

$R^1$ und $R^2$ zusammen die Gruppe $-CH_2CH_2CH_2-$ bedeuten, wobei im letzteren Fall die Bindung an den Sechsring über die C-Atome in 5- und 6-Position des Kerns erfolgt, dadurch gekennzeichnet, dass man eine aromatische Verbindung der Formel II

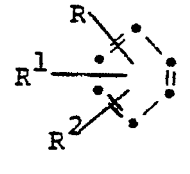

(II)

in der an mindestens 2 benachbarten C-Atomen des Kerns die H-Atome nicht substituiert sind und in der R, $R^1$ und $R^2$ dieselbe Bedeutung wie in Formel I haben, entweder zusammen mit einem Dihalogenmaleinsäureanhydrid der Formel III

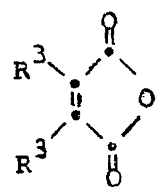

(III)

oder zusammen mit einem Ester der Formel IV

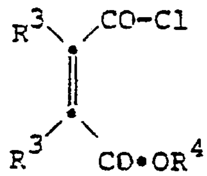

(IV)

wobei $R^3$ dieselbe Bedeutung wie in Formel I hat und $R^4$ jeweils einen niedermolekularen organischen Rest, insbesondere einen aliphatischen, einen cycloaliphatischen, einen aliphatisch-aromatischen oder einen aromatischen Rest, bedeutet, jeweils im etwa stöchiometrischen Mengenverhältnis, in Gegenwart von $AlCl_3$, gegebenenfalls in Gegenwart von inerten Schmelz- und/oder Lösungsmitteln, bei Temperaturen zwischen 40° und 150 °C einer Kondensationsreaktion unterwirft und das so erhaltene Reaktionsprodukt hydrolysiert.

2. Verfahren nach Anspruch 1, dadurch gekennzeichnet, dass man die Kondensationsreaktion in der Weise 2-stufig durchführt, dass man in dem Reaktionsgemisch in der 1. Stufe in an sich bekannter Weise eine Ketocarbonsäure der Formel V

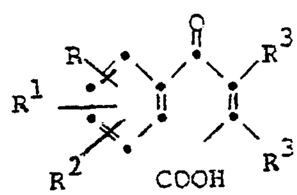

(V)

herstellt und dieselbe isoliert und, dass man in der 2. Stufe die Ketosäure der Formel V ebenfalls in Gegenwart von $AlCl_3$, gegebenenfalls in Gegenwart von inerten Schmelz- und/oder Lösungsmitteln, aber bei Temperaturen zwischen 40° und 150 °C zur entsprechenden Indenoncarbonsäure der Formel I cyclisiert, welche durch Hydrolyse aus dem entstandenen Reaktionsgemisch freigesetzt wird.

3. Verfahren nach Anspruch 1, dadurch gekennzeichnet, dass man Dihalogenmaleinsäureanhydride oder Ester der Formeln III bzw. IV, in denen $R^3$ für Chlor steht, einsetzt.

4. Verfahren nach Anspruch 1, dadurch gekennzeichnet, dass man einen Ester der Formel IV einsetzt, in der $R^4$ einen aliphatischen Rest mit insgesamt 1 bis 4 C-Atomen, vorzugsweise-$CH_3$, bedeutet.

5. Verfahren nach Anspruch 1, dadurch gekennzeichnet, dass man $AlCl_3$ in einer Menge von mindestens 1 Mol pro Mol des Dihalogenmaleinsäureanhydrids der Formel III oder des Esters der Formel IV einsetzt.

6. Verfahren nach Anspruch 1, dadurch gekennzeichnet, dass man die Kondensation in Gegenwart eines inerten Lösungsmittels, vorzugsweise 1,1,2,2-Tetrachloräthan oder Nitrobenzol, durchführt.

7. Verfahren nach Anspruch 6, dadurch gekennzeichnet, dass man die Kondensation in Gegenwart von Dialkyl-amiden niedermolekularer Carbonsäuren als Lösungsmittel, vorzugsweise in Gegenwart von dimethylformamid, durchführt, wobei das Molverhältnis Dialkylamid zu $AlCl_3$ mindestens 1 : 1 beträgt.

8. Verfahren nach Anspruch 1, dadurch gekennzeichnet, dass man die Kondensation in Gegenwart von inerten Schmelzmitteln ablaufen lässt, wobei die Mengen vorzugsweise so gross sind, dass im Gemisch mit dem $AlCl_3$ in der Reaktionsmischung ein Schmelzpunkt resultiert, welcher niedriger als die Reaktionstemperatur ist.

9. Verfahren nach Anspruch 8, dadurch gekennzeichnet, dass man als Schmelzmittel anorganische Salze oder organische Schmelzmittel oder Gemische der Salze mit den organischen Schmelzmitteln einsetzt, wobei die Mengen vorzugsweise so gross sind, dass im Gemisch mit dem $AlCl_3$ in der Reaktionsmischung eine Schmelzpunkterniedrigung der Salzmischung resultiert.

10. Verfahren nach Anspruch 9, dadurch gekennzeichnet, dass man als organische Schmelzmittel Dialkylamide niedermolekularer Carbonsäuren, vorzugsweise Dimethylformamid, einsetzt, wobei das Molverhältnis Dialkylamid zu $AlCl_3$ vorzugsweise in den Grenzen 1 : 4 bis 1 : 1 liegt.

11. Verfahren nach Anspruch 9, dadurch gekennzeichnet, dass man als anorganische Salze NaCl und/oder KCl, gegebenenfalls zusammen mit Dimethylformamid als organisches Schmelzmittel, einsetzt.

12. Verbindungen der Formel VI

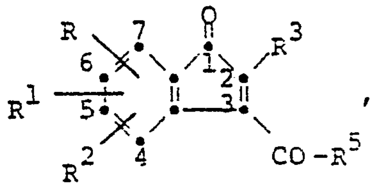 (VI)

worin

$R^3$ für Cl oder Br, vorzugsweise für Cl, steht und

R, $R^1$ und $R^2$ gleich oder verschieden sind,

R einen n-Alkylrest mit 1 bis 4 C-Atomen, vorzugsweise $CH_3$, oder aber H, Cl, Br oder F,

$R^1$ einen n-Alkylrest mit 1 bis 4 C-Atomen, vorzugsweise $CH_3$, oder aber H,

$R^2$ H oder $CH_3$ oder aber

$R^1$ und $R^2$ zusammen die Gruppe $-CH_2CH_2CH_2-$ bedeuten, wobei im letzteren Fall die Bindung an den Sechsring über die C-Atome in 5- und 6-Position des Kerns erfolgt, worin

$R^5$ einen der Reste

$-OH$,

$-O-(CH_2)_m-OH$,

$-O-(CH_2-CH_2-O)_n-CH_2-CH_2-CH$,

$-O-(CH_2)_m-O \cdot CO \cdot \underset{R^6}{\underset{|}{C}} = CH_2$,

$-O-CH_2-\underset{OH}{\underset{|}{CH}}-CH_2-O \cdot CO-\underset{R^6}{\underset{|}{C}} = CH_2$,

$-O-CH_2-CH_2-O-CH = CH_2$ und

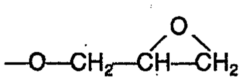

bedeutet,

wobei m eine Zahl von 2 bis 6, n eine Zahl von 1 bis 10 und $R^6$ H oder $-CH_3$ darstellen, mit der Massgabe, dass in dem Fall, in dem $R^5$ $-OH$ bedeutet, mindestens ein Wasserstoffatom in 4- bis 7-Stellung des Sechsrings substituiert ist.

13. Halogen-indenoncarbonsäuren nach Anspruch 12, dadurch gekennzeichnet, dass $R^5$ in Formel VI $-OH$ bedeutet.

14. Verbindungen nach Anspruch 12, dadurch gekennzeichnet, dass sie der Formel VII

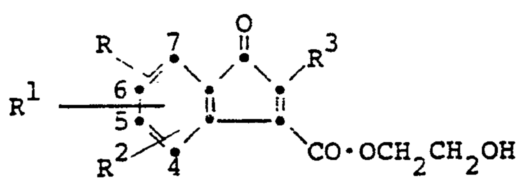 (VII)

entsprechen, in der die Reste R bis $R^3$ dieselbe Bedeutung wie in Formel VI haben.

15. Verbindungen nach Anspruch 12, dadurch gekennzeichnet, dass sie der Formel VIII

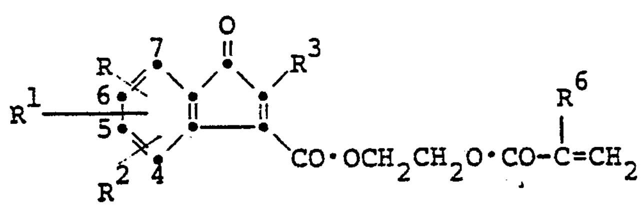 (VIII)

entsprechen, in der die Reste R bis $R^6$ dieselbe Bedeutung wie in Formel VI haben.

16. Verbindungen nach Anspruch 12, dadurch gekennzeichnet, dass sie der Formel XVII

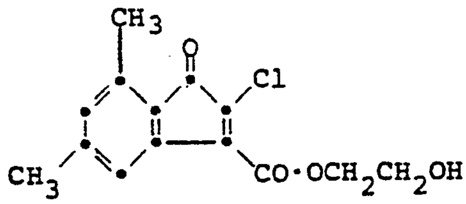

(XVII)

entsprechen.

17. Verbindungen nach Anspruch 12, dadurch gekennzeichnet, dass sie der Formel XVIII

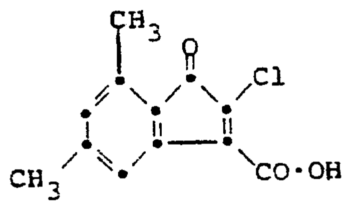

(XVIII)

entsprechen.

18. Verbindungen nach Anspruch 12, dadurch gekennzeichnet, dass sie der Formel XIX

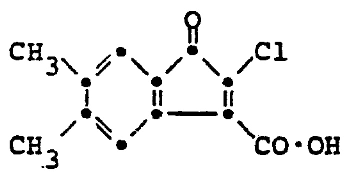

(XIX)

entsprechen.

**Claims**

1. A process for the preparation of an indenoncarboxylic acid of the formula I

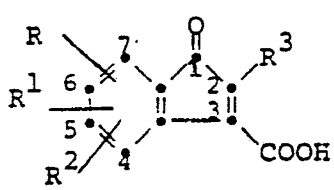

(I)

in which

$R^3$ is Cl or Br, preferably Cl, and

R, $R^1$ and $R^2$ are identical or different and

R is a n-alkyl radical having 1 to 4 C atoms, preferably $CH_3$, or is H, Cl, Br or F,

$R^1$ is a n-alkyl radical having 1 to 4 C atoms, preferably $CH_3$, or is H, and

$R^2$ is H or $CH_3$, or

$R^1$ and $R^2$ together are the group $—CH_{C}H_2CH_2—$, in which latter case the bond to the six-membered ring is via the C atoms in the 5-position and 6-position of the nucleus, which comprises subjecting an aromatic compound of the formula II

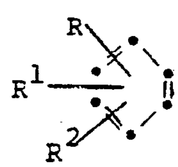

(II)

in which the H atoms on at least 2 adjacent C atoms of the nucleus have not been replaced by substituents and in which R, $R^1$ and $R^2$ are as defined for formula I, either together with a dihalogenomaleic anhydride of the formula III

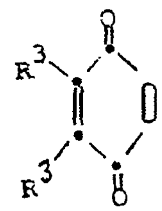

(III)

or together with an ester of the formula IV

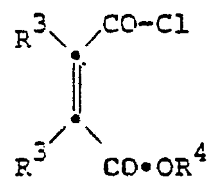

(IV)

in which formulae $R^3$ is as defined for formula I and $R^4$ is in each case a low-molecular organic radical, especially an aliphatic radical, a cycloaliphatic radical, an aliphatic-aromatic radical or an aromatic radical, in each case in an approximately stoichiometric ratio, to a condensation reaction in the presence of $AlCl_3$ and if desired in the presence of inert fluxes and/or solvents, at temperatures between 40° and 150 °C, and hydrolysing the reaction product thus obtained.

2. A process according to claim 1, wherein the condensation reaction is carried out in 2 stages by preparing a keto-carboxylic acid of the formula V

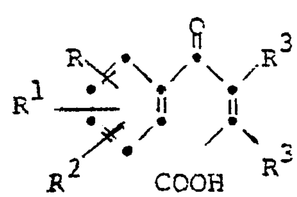

(V)

in the reaction mixture in the 1st stage, in a manner known per se, and isolating this acid and, in the 2nd stage, cyclising the ketoacid of the formula V, likewise in the presence of $AlCl_3$ and if desired in the presence of inert fluxes and/or solvents, but at temperatures between 40° and 150 °C, to give the corresponding indenoncarboxylic acid of the formula I, which is liberated from the resulting reaction mixture by hydrolysis.

3. A process according to claim 1, wherein a dihalogenomaleic anhydride or ester of the formula III or IV is used in which $R^3$ is chlorine.

4. A process according to claim 1, wherein an ester of the formula IV is used in which $R^4$ is an aliphatic radical having a total of 1 to 4 C atoms, preferably $—CH_3$.

5. A process according to claim 1, wherein $AlCl_3$ is used in an amount of at least 1 mol per mol of the dihalogenomaleic anhydride of the formula III or of the ester of the formula IV.

6. A process according to claim 1, wherein the condensation reaction is carried out in the presence of an inert solvent, preferably 1,1,2,2-tetrachloroethane or nitrobenzene.

7. A process according to claim 6, wherein the condensation reaction is carried out in the presence of a dialkylamide of a low-molecular carboxylic acid as the solvent, preferably in the presence of dimethylformamide, the molar ratio of diakylamide to $AlCl_3$ being at least 1 : 1.

8. A process according to claim 1, wherein the condensation reaction is allowed to proceed in the presence of inert fluxes, the amounts preferably being such that the melting point which results when the fluxes are mixed with the $AlCl_3$ in the reaction mixture is lower than the reaction temperature.

9. A process according to claim 8, wherein the fluxes used are inorganic salts or organic fluxes or mixtures of the salts with the organic fluxes, the amounts preferably being such that a lowering of the melting point of the salt mixture results when the fluxes are mixed with the $AlCl_3$ in the reaction mixture.

10. A process according to claim 9, wherein the organic fluxes used are dialkylamides of low-molecular carboxylic acids, preferably dimethylformamide, the molar ratio of dialkylamide to $AlCl_3$ preferably being within the limits 1 : 4 to 1 : 1.

11. A process according to claim 9, wherein the inorganic salts employed are NaCl and/or KCl, if desired together with dimethylformamide as an organic flux.

12. A compound of the formula VI

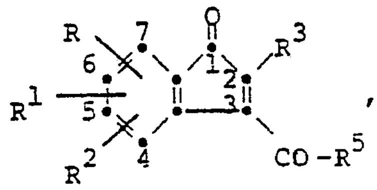

(VI)

in which
$R^3$ is Cl or Br, preferably Cl, and
R, $R^1$ and $R^2$ are identical or different and
R is a n-alkyl radical having 1 to 4 C atoms, preferably $CH_3$, or is H, Cl, Br or F,
$R^1$ is a n-alkyl radical having 1 to 4 C atoms, preferably $CH_3$, or is H, and
$R^2$ is H or $CH_3$, or
$R^1$ and $R^2$ together are the group $-CH_2CH_2CH_2-$, in which latter case the bond to the six-membered ring is via the C atoms in the 5-position and 6-position of the nucleus, and in which
$R^5$ is one of the radicals

$-OH$,
$-O-(CH_2)_m-OH$,
$-O-(CH_2-CH_2-O)_n-CH_2-CH_2-OH$,
$-O-(CH_2)_m-O \cdot CO \cdot C(R^6)=CH_2$,
$-O-CH_2-CH(OH)-CH_2-O \cdot CO-C(R^6)=CH_2$,
$-O-CH_2-CH_2-O-CH=CH_2$ and

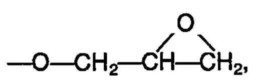

in which m is a number from 2 to 6, n is a number from 1 to 10 and $R^6$ is H or $-CH_3$, with the proviso that if $R^5$ is $-OH$ at least one hydrogen atom in the 4-position to 7-position of the six-membered ring has been replaced by a substituent.

13. A halogeno-indenonecarboxylic acid according to claim 12, wherein $R^5$ in formula VI is $-OH$.

14. A compound according to claim 12, which has the formula VII

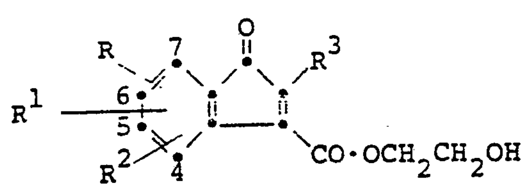

(VII)

in which the radicals R to $R^3$ are as defined for formula VI.

15. A compound according to claim 12, which has the formula VIII

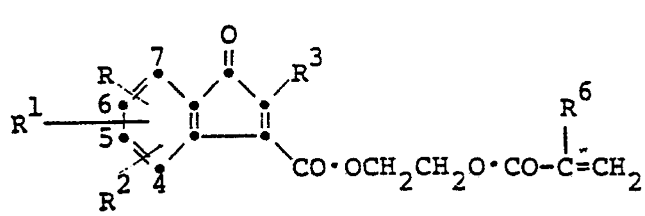

(VIII)

in which the radicals R to $R^6$ are as defined for formula VI.

16. A compound according to claim 12, which has the formula XVII

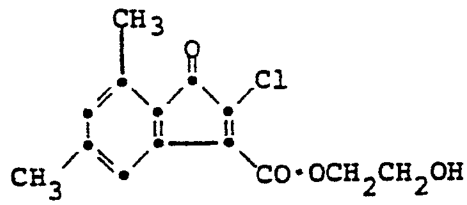

(XVII)

17. A compound according to claim 12, which has the formula XVIII

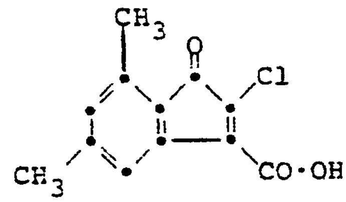

(XVIII)

18. A compound according to claim 12, which has the formula XIX

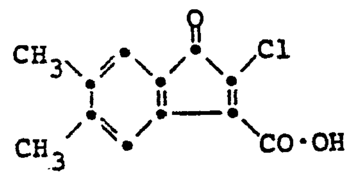

(XIX)

**Revendications**

1. Procédé de préparation d'acides indénone-carboxyliques qui répondent à la formule (I) :

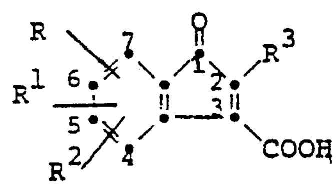

(I)

dans laquelle :

$R^3$ représente Cl ou Br, de préférence Cl,

R, $R^1$ et $R^2$ sont identiques ou différents et ont les significations suivantes :

R représente un radical alkyle contenant de 1 à 4 atomes de carbone, de préférence $CH_3$ ou encore H, Cl, Br ou F,

$R^1$ représente un radical n-alkyle contenant de 1 à 4 atomes de carbone, de préférence $CH_3$, ou encore H,

$R^2$ représente H ou $CH_3$, ou

$R^1$ et $R^2$ forment ensemble un groupement $—CH_2CH_2CH_2—$ et, dans ce dernier cas, la liaison au noyau hexagonal se fait par les atomes de carbone des positions 5 et 6 du noyau, procédé caractérisé en ce qu'on soumet à une réaction de condensation un composé aromatique répondant à la formule (II) :

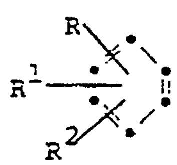

(II)

dans laquelle au moins 2 atomes de carbone en ortho du noyau sont dépourvus de substituant (donc portent des atomes d'hydrogène) et dans laquelle R, $R^1$ et $R^2$ ont les mêmes significations que dans la formule (I), soit avec un anhydride d'acide dihalogéno-maléique répondant à la formule (III) :

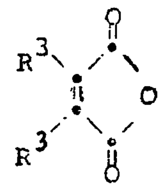

(III)

soit avec un ester répondant à la formule (IV) :

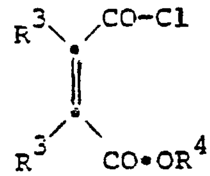

(IV)

formules dans lesquelles $R^3$ a la même signification que dans la formule (I) et $R^4$ représente un radical organique à bas poids moléculaire, plus particulièrement un radical aliphatique, cycloaliphatique, aliphatique-aromatique ou aromatique, à chaque fois dans un rapport à peu près stochiométrique, en présence d'$AlCl_3$, le cas échéant en présence de fondants et/ou de solvants inertes, à des températures comprises entre 40 et 150 °C, et on hydrolyse le produit réactionnel ainsi obtenu.

2. Procédé selon la revendication 1, caractérisé en ce qu'on effectue la réaction de condensation en deux étapes, c'est-à-dire qu'on prépare dans le mélange réactionnel, lors de la première étape, de manière connue, un acide carboxylique cétonique de formule (V) :

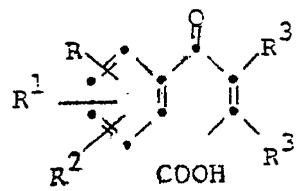

(V)

et on l'isole et, dans la seconde étape, on cyclise l'acide cétonique de fomule (V), également en présence d'$AlCl_3$, éventuellement en présence de fondants et/ou de solvants inertes, mais à des températures comprises entre 40 et 150 °C, pour obtenir l'acide indénone-carboxylique de formule (I) correspondant, qu'on met en liberté par hydrolyse à partir du mélange réactionnel formé.

3. Procédé selon la revendication 1, caractérisé en ce qu'on utilise des anhydrides ou des esters d'acides dihalogénomaléiques de formules (III) ou (IV) dans lesquels $R_3$ représente le chlore.

4. Procédé selon la revendication 1, caractérisé en ce qu'on utilise un ester de formule (IV) dans lequel $R^4$ représente un radical aliphatique contenant au total de 1 à 4 atomes de carbone, de préférence $-CH_3$.

5. Procédé selon la revendication 1, caractérisé en ce qu'on utilise $AlCl_3$ en une quantité d'au moins 1 mole par mole de l'anhydride dihalogéno-maléique de formule (III) ou de l'ester de formule (IV).

6. Procédé selon la revendication 1, caractérisé en ce qu'on effectue la condensation en présence d'un solvant inerte, de préférence de tétrachloro-1,1,2,2 éthane ou de nitrobenzène.

7. Procédé selon la revendication 6, caractérisé en ce qu'on effectue la condensation en présence de dialkylamides d'acides carboxyliques à bas poids moléculaire comme solvants, de préférence en présence de diméthylformamide, le rapport molaire du dialkylamide à $AlCl_3$ étant d'au moins 1 : 1.

8. Procédé selon la revendication 1, caractérisé en ce qu'on effectue la condensation en présence de fondants inertes en quantités de préférence suffisantes pour qu'il en résulte, en mélange avec $AlCl_3$ dans le mélange réactionnel, un point de fusion inférieur à la température de réaction.

9. Procédé selon la revendication 8, caractérisé en ce qu'on emploie comme fondants des fondants minéraux ou organiques ou des mélanges de sels avec des fondants organiques en quantités de préférence suffisantes pour que, en mélange avec $AlCl_3$, il en résulte dans le mélange réactionnel un abaissement du point de fusion du mélange de sels.

10. Procédé selon la revendication 9, caractérisé en ce qu'on utilisé, comme fondants organiques, des dialkylamides d'acides carboxyliques à bas poids moléculaire, de préférence le diméthylformamide, le rapport molaire du dialkylamide à $AlCl_3$ étant de préférence compris entre 1 : 4 et 1 : 1.

11. Procédé selon la revendication 9, caractérisé en ce qu'on utilise, comme sels minéraux, NaCl et/ou KCl, éventuellement avec du diméthylformamide en tant que fondant organique.

12. Composés répondant à la formule (VI) :

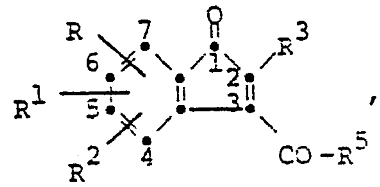

(VI)

dans laquelle :

$R^3$ représente Cl ou Br, de préférence Cl,

R, $R^1$ $R^2$ sont identiques ou différents et ont les significations suivantes :

R représente un radical n-alkyle contenant de 1 à 4 atomes de carbone, de préférence $-CH_3$, ou encore H, Cl, Br ou F,

$R^1$ représente un radical n-alkyle contenant de 1 à 4 atomes de carbone, de préférence $-CH_3$ ou encore H,

$R^2$ représente H ou $-CH_3$, ou

$R^1$ et $R^2$ forment ensemble un groupement $-CH_2CH_2CH_2-$ qui est relié au noyau hexagonal par les atomes de carbone des positions 5 et 6 de celui-ci, et

$R^5$ représente l'un des radicaux suivants :

$-OH$,

$-O-(CH_2)_m-OH$,

$-O-(CH_2-CH_2-O)_n-CH_2-CH_2-OH$,

$-O-(CH_2)_m-O \cdot CO \cdot \underset{R^6}{\underset{|}{C}} = CH_2$,

$-O-CH_2-\underset{OH}{\underset{|}{CH}}-CH_2-O \cdot CO-\underset{R^6}{\underset{|}{C}} = CH_2$,

$-O-CH_2-CH_2-O-CH = CH_2$ et

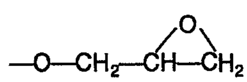

dans lesquels m désigne un nombre de 2 à 6, n un nombre de 1 à 10 et $R^6$ représente H ou $-CH_3$, avec la condition que, dans le cas où $R^5$ représente $-OH$, au moins un atome d'hydrogène des positions 4 à 7 du noyau hexagonal soit remplacé par un substituant.

13. Acides halogéno-indénone-carboxyliques selon la revendication 12, caractérisés en ce que, dans la formule (VI), $R^5$ représente $-OH$.

14. Composés selon la revendication 12, caractérisés en ce qu'ils répondent à la formule (VII) :

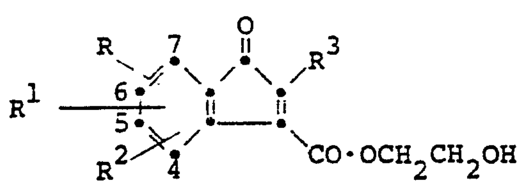

(VII)

dans laquelle les symboles R à $R^3$ ont les mêmes significations que dans la formule (VI).

15. Composés selon la revendication 12, caractérisés en ce qu'ils répondent à la formule (VIII) :

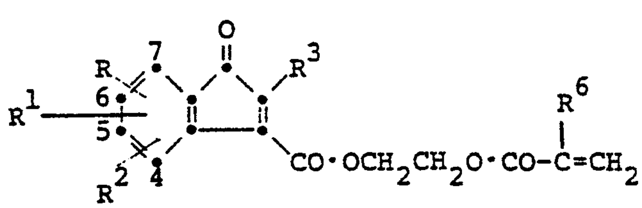

(VIII)

dans laquelle les symboles R à $R^6$ ont les mêmes significations que dans la formule (VI).

16. Composé selon la revendication 12, qui répond à la formule (XVII) :

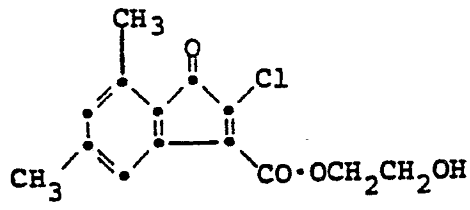

(XVII)

17. Composé selon la revendication 12 caractérisé en ce qu'il répond à la formule (XVIII) :

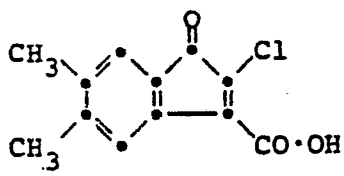

(XVIII)

18. Composé selon la revendication 12 caractérisé en ce qu'il répond à la formule (XIX).

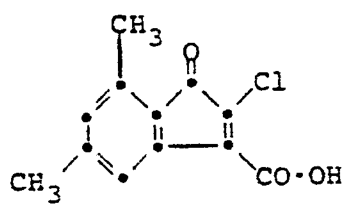

(XIX)